# NEUE EUROPÄISCHE PATENTSCHRIFT

(11) **EP 3 071 135 B2**
(45) Veröffentlichungstag und Bekanntmachung des Hinweises auf die Entscheidung über den Einspruch: **30.09.2020**
(45) Hinweis auf die Patenterteilung: 01.11.2017
(21) Anmeldenummer: 14798887.7
(22) Anmeldetag: 14.11.2014
(51) Int. Cl.: A61B 18/12, A61B 18/00

(54) **HOCHFREQUENZ-CHIRURGIEGERÄT UND VERFAHREN ZUM BETREIBEN EINES SOLCHEN**
HIGH-FREQUENCY SURGICAL APPLIANCE AND METHOD FOR OPERATING SUCH AN APPLIANCE
APPAREIL CHIRURGICAL À HAUTE FRÉQUENCE ET PROCÉDÉ POUR LE FAIRE FONCTIONNER

(30) Priorität: 19.11.2013 DE 102013223561
(43) Veröffentlichungstag der Anmeldung: 28.09.2016
(73) Patentinhaber: Olympus Winter & Ibe GmbH, 22045 Hamburg (DE)
(72) Erfinder: STEIN, Thomas, 14513 Teltow (DE)
(74) Vertreter: Eisenführ Speiser
(86) Internationale Anmeldenummer: PCT/EP2014/074683
(87) Internationale Veröffentlichungsnummer: WO 2015/074972

(56) Entgegenhaltungen:
- EP-A1- 0 219 568
- EP-A1- 0 316 469
- DE-A1- 4 126 608
- DE-A1- 4 217 999
- DE-B2- 2 504 280

## Beschreibung

Die Erfindung betrifft ein Hochfrequenz-Chirurgiegerät zum Schneiden und/oder Koagulieren von biologischem Gewebe mit einem Hochfrequenz-Generator, der ausgebildet ist, im Betrieb einen hochfrequenten Wechselstrom zu erzeugen.

Unter dem Begriff Hochfrequenz-Chirurgie versteht man im Wesentlichen das Schneiden und/oder Koagulieren (Veröden) von biologischem Gewebe unter Verwendung hochfrequenten Wechselstroms (vorzugsweise ca. 0,2MHz oder 0,3MHz bis 3MHz, teilweise bis zu 5MHz). Dazu werden vorzugsweise Hochfrequenz-Chirurgiesysteme mit einem Hochfrequenz-Chirurgiegerät und einem daran angeschlossenen elektrochirurgischen Instrument eingesetzt.

Bei der Koagulation wird hochfrequenter Wechselstrom insbesondere zur Blutstillung oder zur Ablation von Gewebe eingesetzt. Hierbei wird Gewebe, welches eine Elektrode eines elektrochirurgischen Instruments umgibt, soweit erwärmt, dass es zur Denaturierung und einem Zusammenschrumpfen des Gewebes und der Blutgefäße, und damit letztlich zur Stillung von Blutungen kommt. Auch bei der Ablation wird ein Gewebebereich auf diese Weise denaturiert. Der so behandelte Gewebebereich vernarbt und wird durch körpereigene Prozesse abgebaut, ohne dass er operativ entfernt werden muss.

Beim Schneidvorgang beruht der Schneideffekt in biologischem Gewebe auf einer Lichtbogenbildung zwischen einer aktiven Elektrode, der sogenannten Schneidelektrode, eines elektrochirurgischen Instruments und dem Gewebe. Elektrisch leitfähiges Gewebe kann dabei nahezu ohne mechanischen Druck durchtrennt werden.

Bei der monopolaren Technik werden eine aktive Koagulations- oder Schneidelektrode eines elektrochirurgischen Instruments und eine großflächig aufgebrachte Neutralelektrode verwendet. Der Hochfrequenzstrom fließt dabei von der aktiven Elektrode über das zu behandelnde Gewebe zur Neutralelektrode. Entscheidend für die thermische Wirkung des Stroms an der Applikationsstelle ist eine kleinflächige aktive Elektrode gegenüber einer großflächigen Neutralelektrode. Dadurch werden eine hohe Stromdichte und damit eine starke Erwärmung des Gewebes an der Operationsstelle erreicht und gleichzeitig ungewollte Gewebeschädigungen an der neutralen Elektrode vermieden.

Bei bipolaren Anwendungen werden zwei Elektroden verwendet, die in einem elektrochirurgischen Instrument zusammengefasst oder an unterschiedlichen elektrochirurgischen Geräten ausgebildet sind. Der Hochfrequenzstrom fließt dabei von der einen Elektrode über das zu behandelnde Gewebe zur anderen Elektrode des elektrochirurgischen Instruments.

Aus der Offenlegungsschrift DE 42 17 999 ist ein Hochfrequenz-Chirurgiegerät zum Schneiden biologischen Gewebes mit einem Hochfrequenz-Leistungsgenerator und einer Einstelleinrichtung bekannt, an welcher die Hochfrequenz-Ausgangsleistung einstellbar ist sowie mit einer Einrichtung zum Aktivieren des Hochfrequenz-Leistungsgenerators und einer Einrichtung, welche nach jedem Aktivieren des Hochfrequenz- Leistungsgenerators zeitlich limitiert eine höhere HF-Ausgangsleistung zulässt als die an der Einstelleinrichtung jeweils eingestellte Hochfrequenz-Ausgangsleistung. Zudem ist eine Spannungs-Sensoreinrichtung zur automatischen Überwachung des Ist-Werts der HF-Ausgangsspannung und/oder eine Lichtbogen-Sensoreinrichtung zur Überwachung der Intensität der elektrischen Lichtbogen zwischen der aktiven Elektrode und dem Gewebe vorgesehen. Der Ist-Wert der HF-Ausgangsspannung und/oder der Intensität der elektrischen Lichtbogen wird mit einem Soll-Wertl der HF-Spannung beziehungsweise der Ist-Wert der Intensität der elektrischen Lichtbogen mit einem Soll-Wert der Intensität der elektrischen Lichtbogen verglichen.

Die europäische Patentanmeldung EP 0 219 568 offenbart ein Hochfrequenz-Chirurgiegerät mit automatischer Regelung der Intensität der elektrischen Lichtbogen zwischen der aktiven Elektrode und dem zu schneidenden oder zu koagulierenden Gewebe, wobei zur Feststellung des Ausmaßes der Lichtbogen ein davon abhängiges elektrisches Signal über ein Filter abgeleitet und einem Gleichrichter zugeführt wird, dessen Ausgangssignal einem Regelverstärker zugeführt wird, in welchem dieses Ausgangssignal entweder mit dem Sollwertsignal eines Sollwertgebers für den Schneidevorgang oder mit dem Sollwert eines Sollwertgebers für den Koagulationsvorgang verglichen wird. Der Regelverstärker erzeugt ein der Differenz zweier Signale proportionales Signal, welches einem Amplitudenmodulator zugeführt wird, welcher die Amplitude der Ausgangsspannung des Leistungsverstärkers steuert. Das Filter ist derart ausgebildet, dass es mindestens eine Frequenz der von den elektrischen Lichtbogen zwischen aktiver Elektrode und Gewebe erzeugten nichtharmonischen Frequenzen der Grundfrequenz des Hochfrequenz-Oszillators aus der elektrischen Spannung oder aus dem elektrischen Strom im Ausgang durchlässt und gleichzeitig sowohl die Grundfrequenz als auch deren harmonische Frequenzen so stark dämpft, dass sie keinen Einfluss auf das Regelsignal haben.

Die Offenlegungsschrift DE41 26 608 offenbart eine Anordnung zum Schneiden von biologischem Gewebe mit Hochfrequenzstrom, bei der die an das Gewebe abgegebene Leistung auf das momentan benötigte Maß eingestellt werden kann, wenn das Ausmaß des beim Schneiden zwischen Chirurgiesonde und Gewebe brennenden Lichtbogens konstant geregelt wird.

Hochfrequenz-Chirurgiegeräte der eingangs genannten Art sind aus dem Stand der Technik bekannt und dienen dazu, elektrochirurgische Instrumente mit Hochfrequenzstrom zu versorgen. Sie werden in der Chirurgie für unterschiedliche Prozeduren und Behandlungen eingesetzt. Für die unterschiedlichen Anwendungsgebiete und beispielsweise aufgrund der je nach Gewebe deutlich unterschiedlichen Gewebeimpedanzen können Hochfrequenz-Chirurgiegeräte mit unterschiedlicher Ausgangsleistung betrieben werden. Gleichzeitig wird bei vielen Hochfrequenz-Chirurgiegeräten mittels einer sogenannten Funkenregelung die Funkenintensität begrenzt und konstant gehalten. Ziel ist es, für Patienten und Anwender sichere und effiziente Behandlungen durchzuführen und insbesondere reproduzierbare Schneide- und/oder Koagulationseffekte zu erzielen. Eine Anforderung an Hochfrequenz-Chirurgiegeräte ist es daher, reproduzierbare Schneid- und/oder Koagulationseigenschaften, weitgehend unabhängig von äußeren Bedingungen, wie z. B. der Schnittgeschwindigkeit oder Elektrodengröße, sicherzustellen. Beim Einsatz elektrochirurgischer Systeme kann es jedoch in der Praxis vorkommen, dass Elektroden am Gewebe festkleben oder die Schneid- und/oder Koagulationsqualität unzureichend ist. Dies kann zu unerwünschten Behandlungsunterbrechungen führen und/oder die Behandlungszeit verlängern.

Es ist daher eine Aufgabe der vorliegenden Erfindung, ein Hochfrequenz-Chirurgiegerät bereitzustellen, welches existierende Hochfrequenz-Chirurgiegeräte verbessert. Insbesondere ist es eine Aufgabe der vorliegenden Erfindung, ein Hochfrequenz-Chirurgiegerät bereitzustellen, welches die Anzahl und/oder Dauer von Behandlungsunterbrechungen und damit vorzugsweise auch die Behandlungszeit reduzieren kann.

Diese Aufgabe wird erfindungsgemäß gelöst durch ein gemäß Anspruch 1 zum Schneiden und/oder Koagulieren von biologischem Gewebe, mit einem Hochfrequenz-Generator, der ausgebildet ist, im Betrieb einen hochfrequenten Wechselstrom zu erzeugen. Das Hochfrequenz-Chirurgiegerät weist ferner eine Leistungseinstelleinrichtung auf, die angeordnet und ausgebildet ist, eine Ausgangsleistung des Hochfrequenz-Chirurgiegeräts auf einen von einem Anwender ausgewählten Leistungswert einzustellen bzw. zu begrenzen. Ferner ist eine Funkenkontrolleinrichtung vorgesehen, die angeordnet und ausgebildet ist, eine Funkenspannung auf einen Soll-Funkenwert einzustellen bzw. zu begrenzen, wobei die Funkenkontrolleinrichtung ferner ausgebildet ist, den Soll-Funkenwert in Abhängigkeit von dem von einem Anwender ausgewählten Leistungswert zu ermitteln.

Hochfrequenz-Chirurgiegeräte zum Schneiden und/oder Koagulieren von biologischem Gewebe weisen einen Hochfrequenz-Generator auf, der hochfrequenten Wechselstrom erzeugt. Über eine Leistungseinstelleinrichtung kann die Ausgangsleistung des Hochfrequenz-Chirurgiegeräts von einem Anwender ausgewählt werden. Hierzu ist die Leistungseinstelleinrichtung vorzugsweise mit einer Anwenderschnittstelle verbunden, über die der Anwender, beispielsweise über einen für die Regler, den gewünschten Leistungswert auswählen und vorgeben kann. Die Leistungseinstelleinrichtung ist mit dem Hochfrequenz-Generator vorzugsweise signaltechnisch so verbunden, dass der vom Anwender ausgewählte Leistungswert eingestellt werden kann.

Eine Funkenkontrolleinrichtung sorgt ferner für eine Funkenregelung des Hochfrequenz-Chirurgiegeräts, indem über eine Begrenzung der Funkenspannung auf einen Soll-Funkenwert die Funkenintensität kontrolliert wird.

Bei im Stand der Technik bekannten Hochfrequenz-Chirurgiegeräten mit einer Funkenregelung, welche die Funkenintensität unabhängig von der Leistungseinstellung konstant hält, kann es insbesondere bei niedrigen Funkenintensitäten dazu kommen, dass - obwohl ein Funke vorhanden ist - insbesondere größere, zum Beispiel spateiförmige, Elektroden am Gewebe festkleben oder sich die Schneidqualität bei einer Schneidelektrode verschlechtert. Eine in solchen Fällen häufige Reaktion eines Anwenders, die Leistung über die Leistungseinstellung zu erhöhen hat auf die genannten Nachteile jedoch in den meisten Fällen keinen positiven Einfluss, da die Funkenregelung den Hochfrequenz-Generator weiterhin auf die voreingestellte konstante Funkenintensität begrenzt. Auch unabhängig von den beispielhaft genannten Nachteilen kann es in bestimmten Anwendungsfällen sinnvoll sein, auch bei einem funkengeregelten Hochfrequenz-Chirurgiegerät die Funkenintensität verändern zu können.

Die Erfindung beruht hierbei auf der Erkenntnis, ein Hochfrequenz-Chirurgiegerät vorzusehen, bei dem in einer Funkenkontrolleinrichtung ein Soll-Funkenwert, auf den die Funkenspannung begrenzt wird, in Abhängigkeit des vom Anwender gewählten Leistungswerts ermittelt wird. Hierzu kann in der Kontrolleinrichtung (oder z. B. in einer mit dieser verbundenen Rechen- und/oder Speichereinheit) hinterlegt sein, welche Soll-Funkenwerte für verschiedene, von einem Anwender ausgewählte Leistungswerte zur Begrenzung der Funkenspannung von der Funkenkontrolleinrichtung herangezogen werden sollen. Die Soll-Funkenwerte können beispielsweise in Form einer Kennlinie hinterlegt sein, welche den Soll-Funkenwert in Abhängigkeit des vom Anwender ausgewählten Leistungswertes darstellt.

Durch die Kopplung des Soll-Funkenwerts, auf den die Funkenspannung begrenzt wird, an die vom Anwender ausgewählte und damit vom Anwender beeinflussbare bzw. veränderbare Leistungseinstellung kann erreicht werden, dass auf der einen Seite eine Funkenregelung beibehalten wird und damit die Funkenintensität kontrolliert wird, gleichzeitig aber dem Anwender die Möglichkeit gegeben wird, durch eine Veränderung des Leistungswerts indirekt auch die Funkenintensität zu beeinflussen. Die Einstellung der Ausgangsleistung auf den Leistungswert und die Funkenregelung zur Begrenzung der Funkenspannung auf den Soll-Funkenwert bleiben dabei vorzugsweise unabhängig voneinander und werden lediglich dadurch gekoppelt, dass die Funkenkontrolleinrichtung den Soll-Funkenwert in Abhängigkeit vom Leistungswert ermittelt. Die erfindungsgemäße Ausgestaltung des Hochfrequenz-Chirurgiegeräts ergibt sich dadurch, dass die Funkenkontrolleinrichtung angeordnet und ausgebildet ist, den Soll-Funkenwert derart zu ermitteln, dass der Soll-Funkenwert zumindest in einem bestimmten Wertebereich mit zunehmendem Leistungswert steigt. Hierbei ist vorgesehen, dass einem höheren vom Anwender ausgewählten Leistungswert auch ein höherer Soll-Funkenwert zugeordnet ist und einem entsprechend niedrigeren vom Anwender ausgewählten Leistungswert auch ein niedrigerer Soll-Funkenwert zugeordnet ist bzw. ein entsprechend niedrigerer Soll-Funkenwert von der Funkenkontrolleinrichtung ermittelt wird. Auf diese Weise kann die intuitive Anwendererwartung, dass mit steigendem Leistungswert auch die Funkenintensität zunimmt, in der Funkenkontrolleinrichtung abgebildet werden, so dass sich für den Anwender eine besonders einfache Bedienung ergibt.

Diese Kopplung eines steigenden Soll-Funkenwerts an einen zunehmenden Leistungswert ist vorzugsweise mindestens in einem bestimmten Wertebereich vorgesehen. Diese Kopplung kann jedoch auch über den gesamten Ausgangsleistungsbereich des Hochfrequenz-Chirurgiegeräts ausgebildet sein. Alternativ können in verschiedenen Wertebereichen der gesamten Spanne der Ausgangsleistung des Hochfrequenz-Chirurgiegeräts unterschiedliche Kopplungen des Soll-Funkenwerts an den Leistungswert vorgesehen sein. Beispielsweise kann in einem bestimmten Wertebereich, vorzugsweise am unteren Ende der Spanne der Ausgangsleistung des Hochfrequenz-Chirurgiegeräts, vorgesehen sein, dass der Soll-Funkenwert auch bei zunehmendem Leistungswert konstant bleibt und erst in einem Wertebereich höherer Ausgangsleistung mit zunehmenden Leistungswert ansteigt.

In einer weiteren Ausgestaltung des Hochfrequenz-Chirurgiegerät ist bevorzugt, dass die Funkenkontrolleinrichtung angeordnet und ausgebildet ist, den jeweiligen Soll-Funkenwert derart zu ermitteln, dass der Soll-Funkenwert zumindest in einem bestimmten Wertebereich proportional mit dem Leistungswert steigt.

Eine proportionale Kopplung des Soll-Funkenwerts an den Leistungswert hat den Vorteil einer erwartbaren und gleichmäßigen Steigerung der Funkenintensität mit dem Leistungswert. Auch hier ist eine solche Kopplung über die gesamte Spanne der möglichen Ausgangsleistung des Hochfrequenz-Chirurgiegeräts möglich oder in einem oder mehreren bestimmten Wertebereichen innerhalb dieser Gesamtspanne.

Ferner kann vorgesehen sein, dass die Funkenkontrolleinrichtung angeordnet und ausgebildet ist, den Soll-Funkenwert derart zu ermitteln, dass der Soll-Funkenwert einen vorbestimmten Mindest-Funkenwert nicht unterschreitet und/oder einen vorbestimmten Maximal-Funkenwert nicht überschreitet.

Eine Beschränkung des Funkenwerts auf einen maximalen Wert kann aus Sicherheitsgründen bevorzugt sein. Eine Begrenzung des Soll-Funkenwerts auf einen Minimalwert kann bevorzugt sein, wenn sich beispielsweise die Einstellung von Funkenwerten unterhalb des Mindest-Funkenwerts als schwierig oder unzuverlässig darstellt. Dies kann insbesondere bei Soll-Funkenwerten von weniger als 5V der Fall sein, so dass ein Mindestfunkenwert von beispielsweise 5V bevorzugt ist.

Hochfrequenz-Chirurgiegeräte können zwei, drei oder mehrere Betriebsmodi aufweisen. Solche Betriebsmodi können sich in der Einstellung verschiedener Parameter unterscheiden und dienen vorzugsweise dazu, Parametereinstellungen bereitzustellen, die beispielsweise unterschiedliche Schnittgeschwindigkeiten unterstützen. In einem solchen Betriebsmodus können ferner ein, zwei, drei oder mehrere Effektmodi vorgesehen oder von Anwendern auswählbar sein.

Besonders bevorzugt ist es, wenn die Funkenkontrolleinrichtung angeordnet und ausgebildet ist, den Soll-Funkenwert in Abhängigkeit von dem jeweiligen Betriebsmodus und/oder in Abhängigkeit von dem jeweiligen Effektmodus zu ermitteln. Insbesondere können unterschiedliche Kennlinien für den Soll-Funkenwert in Abhängigkeit der verschiedenen Betriebs- bzw. Effektmodi vorgesehen sein.

Ferner sind Hochfrequenz-Chirurgiegeräte bevorzugt, bei denen die Leistungseinstelleinrichtung angeordnet und ausgebildet ist, den Leistungswert so zu begrenzen, dass der Leistungswert einen vorbestimmten Mindest-Leistungswert nicht unterschreitet und/oder einen vorbestimmten Maximal-Leistungswert nicht überschreitet. Die Begrenzung der Ausgangsleistung auf einen Bereich zwischen einem Mindest- und einem Maximalleistungswert kann insbesondere dazu dienen, die Ausgangsleistung auf eine für den Anwendungsbereich erforderliche und gleichzeitig sowohl für den Patienten als auch für den Anwender sicheren Bereich zu begrenzen.

Die Ermittlung des Soll-Funkenwerts durch die Funkenkontrolleinrichtung kann dabei vorzugsweise derart erfolgen, dass einem Mindest-Leistungswert ein bestimmter Soll-Funkenwert zugeordnet ist. Von diesem dem Mindest-Leistungswert zugeordneten Soll-Funkenwert kann dann in vorteilhafter Weise ein beispielsweise proportionaler Anstieg des Soll-Funkenwerts mit dem Anstieg des Leistungswerts erfolgen.

Ferner wird beschrieben ein Verfahren zum Betreiben eines Hochfrequenz-Chirurgiegeräts, insbesondere eines zuvor beschriebenen Hochfrequenz-Chirurgiegeräts bei dem zum Schneiden und/oder Koagulieren von biologischem Gewebe ein hochfrequenter Wechselstrom mittels eines Hochfrequenz-Generators erzeugt wird, wobei mittels einer Leistungseinstelleinrichtung eine Ausgangsleistung des Hochfrequenz-Chirurgiegeräts auf einen von einem Anwender ausgewählten Leistungswert eingestellt wird und mittels einer Funkenkontrolleinrichtung ein Soll-Funkenwert einer Funkenspannung in Abhängigkeit von dem von einem Anwender ausgewählten Leistungswert ermittelt wird und ferner die Funkenspannung mittels der Funkenkontrolleinrichtung auf den Soll-Funkenwert begrenzt wird.

Das Verfahren dient insbesondere dazu, ein Hochfrequenz-Chirurgiegerät wie zuvor beschrieben zu betreiben. Zu den Vorteilen, Ausführungsvarianten und Ausführungsdetails dieses Verfahrens und seiner möglichen Fortbildungen wird auf die vorangegangene Beschreibung zu den entsprechenden Vorrichtungsmerkmalen verwiesen.

Eine bevorzugte Ausführungsform der Erfindung wird beispielhaft anhand der beiliegenden Figuren beschrieben. Es zeigen:
- Figur 1:: eine schematische Darstellung einer beispielhaften Ausführungsform eines erfindungsgemäßen Hochfrequenz-Chirurgiegeräts; und
- Figur 2:: beispielhafte Kennlinien des Soll-Funkenwerts für ein Hochfrequenz-Chirurgiegerät nach Figur 1.

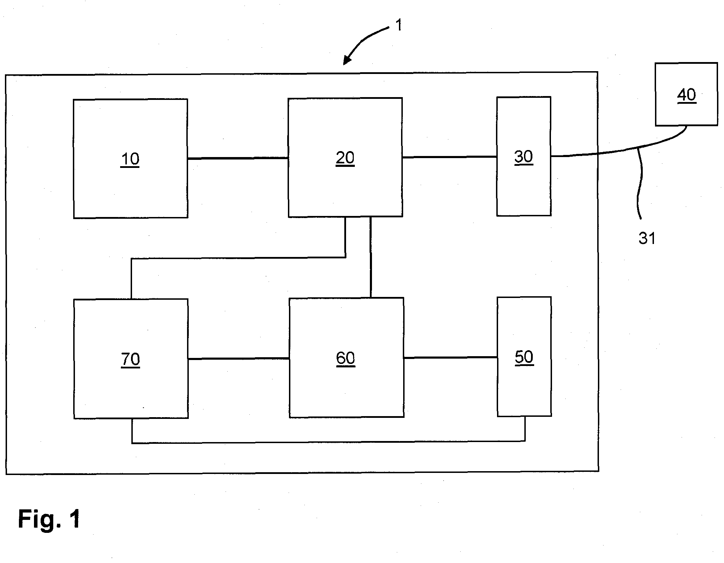

Figur 1 zeigt ein Hochfrequenz-Chirurgiegerät 1 zum Schneiden und/oder Koagulieren von biologischem Gewebe mit einem Hochfrequenz-Generator 20. Der Hochfrequenz-Generator 20 ist mit einem Netzteil 10 verbunden und ausgebildet, im Betrieb einen hochfrequenten Wechselstrom zu erzeugen und über eine Anschlussbuchse 30 an ein über ein Verbindungskabel 31 daran angeschlossenes elektrochirurgisches Instrument 40 abzugeben. Die Ausgangsleistung des Hochfrequenz-Chirurgiegeräts 1 wird über eine Leistungseinstelleinrichtung 60 eingestellt. Über eine Anwenderschnittstelle 50 kann ein Anwender einen gewünschten Leistungswert sowie einen Betriebs- und/oder Effektmodus auswählen, der von der Anwenderschnittstelle 50 an die Leistungseinstelleinrichtung 60 und/oder an die Funkenkontrolleinheit 70 übermittelt wird. In Abhängigkeit von diesem ausgewählten Leistungswert bzw. Betriebs- und/oder Effekt-Modus regelt bzw. begrenzt die Funkenkontrolleinheit 70 die Funkenintensität des Hochfrequenz-Chirungiegeräts 1. Die Leistungseinstelleinrichtung 60 und die Funkenkontrolleinheit 70 sind untereinander und jeweils mit dem Hochfrequenz-Generator 20 und mit der Anwenderschnittstelle 50 verbunden.

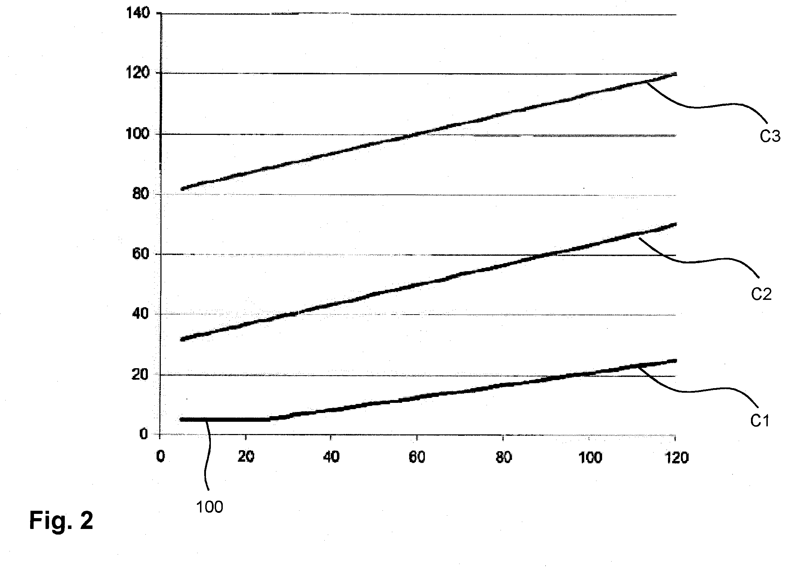

Im Hochfrequenz-Chirurgiegerät 1 ist, vorzugsweise in der Funkenkontrolleinheit 70, hinterlegt, wie der Soll-Funkenwert in Abhängigkeit von den vom Anmelder gewählten Leistungswert ermittelt wird. Hierzu können Zuordnungen in Form von Kennlinien, wie in Figur 2 dargestellt, verwendet werden. Auf der Hochachse in Figur 2 ist die Funkenspannung in Volt aufgetragen, auf der Längsachse die gewählte Leistungsstufe des Hochfrequenz-Chirurgiegeräts in Watt. Die drei Kennlinien C1, C2 und C3 geben Soll-Funkenwerte für drei unterschiedliche Betriebsmodi, hier Schneidmodi, des Hochfrequenz-Chirurgiegeräts 1 vor. Die Kennlinien C2 und C3 verlaufen im dem Wertebereich von einem Mindest-Leistungswert von 5W bis zu einem Maximal-Leistungswert von 120W proportional ansteigend. Die Kennlinie C1 zeigt nur im Wertebereich 25W bis 120W der Ausgangsleistung eine proportionale Kopplung zum Leistungswert. Im Wertebereich von 5W bis 25W weist die Kennlinie C1 einen nicht proportionalen Abschnitt 100 auf, in dem der Soll-Funkenwert konstant bei 5V liegt. Hintergrund hierfür ist, dass insbesondere in einem Bereich unterhalb von 5V eine präzise Einstellung der Funkenspannung schwierig sein kann.

Durch die Hinterlegung bestimmter Kopplungen, beispielsweise in Form von Kennlinien, der Soll-Funkenwerte für bestimmte Leistungswerte kann somit in einfacher Weise einem Anwender die Möglichkeit gegeben werden, über eine Veränderung des Leistungswerts gleichzeitig eine Veränderung der Funkenintensität zu bewirken, ohne auf die Vorteile einer Funkenregelung verzichten zu müssen.

### BEZUGSZEICHEN

- 1: Hochfrequenz-Chirurgiegerät
- 10: Netzteil
- 20: Hochfrequenz-Generator
- 30: Anschlussbuchse
- 31: Verbindungskabel
- 40: Elektrochirurgisches Instrument
- 50: Anwenderschnittstelle
- 60: Leistungseinstelleinrichtung
- 70: Funkenkontrolleinrichtung
- 100: nicht-proportionaler Funkenwert-Bereich
- C1, C2, C3: Betriebsmodi

## Patentansprüche

1. Hochfrequenz-Chirurgiegerät (1) zum Schneiden und/oder Koagulieren von biologischem Gewebe,
- mit einem Hochfrequenz-Generator (20), der ausgebildet ist, im Betrieb einen hochfrequenten Wechselstrom zu erzeugen,
- mit einer Leistungseinstelleinrichtung (60), die angeordnet und ausgebildet ist, eine Ausgangsleistung des Hochfrequenz-Chirurgiegeräts (1) auf einen von einem Anwender ausgewählten Leistungswert einzustellen bzw. zu begrenzen,
- mit einer Funkenkontrolleinrichtung (70), die angeordnet und ausgebildet ist, eine Funkenspannung auf einen Soll-Funkenwert einzustellen bzw. zu begrenzen,
- wobei die Funkenkontrolleinrichtung (70) ferner ausgebildet ist, den Soll-Funkenwert in Abhängigkeit von dem von einem Anwender ausgewählten Leistungswert zu ermitteln, wobei der Soll-Funkenwert derart ermitteln wird, dass der Soll-Funkenwert zumindest in einem bestimmten Wertebereich mit zunehmendem Leistungswert steigt.

2. Hochfrequenz-Chirurgiegerät (1) nach Anspruch 1,
**dadurch gekennzeichnet, dass** die Funkenkontrolleinrichtung (70) angeordnet und ausgebildet ist, den jeweiligen Soll-Funkenwert derart zu ermitteln, dass der Soll-Funkenwert zumindest in einem bestimmten Wertebereich proportional mit dem Leistungswert steigt.

3. Hochfrequenz-Chirurgiegerät (1) nach mindestens einem der beiden vorhergehenden Ansprüche,
**dadurch gekennzeichnet, dass** die Funkenkontrolleinrichtung (70) angeordnet und ausgebildet ist, den Soll-Funkenwert derart zu ermitteln, dass der Soll-Funkenwert einen vorbestimmten Mindest-Funkenwert (100) nicht unterschreitet und/oder einen vorbestimmten Maximal-Funkenwert nicht überschreitet.

4. Hochfrequenz-Chirurgiegerät (1) nach mindestens einem der vorhergehenden Ansprüche,
**dadurch gekennzeichnet, dass** das Hochfrequenz-Chirurgiegerät (1) zwei, drei oder mehrere Betriebsmodi (C1, C2, C3) aufweist, und wobei die Funkenkontrolleinrichtung (70) angeordnet und ausgebildet ist, den Soll-Funkenwert in Abhängigkeit von dem jeweiligen Betriebsmodus (C1, C2, C3) zu ermitteln.

5. Hochfrequenz-Chirurgiegerät (1) nach dem vorhergehenden Anspruch,
**dadurch gekennzeichnet, dass** mindestens einer der Betriebsmodi einen, zwei, drei oder mehrere Effektmodi aufweist, und wobei die Funkenkontrolleinrichtung (70) angeordnet und ausgebildet ist, den Soll-Funkenwert in Abhängigkeit von dem jeweiligen Effektmodus zu ermitteln.

6. Hochfrequenz-Chirurgiegerät (1) nach mindestens einem der vorhergehenden Ansprüche,
**dadurch gekennzeichnet, dass** Leistungseinstelleinrichtung (60) angeordnet und ausgebildet ist, den Leistungswert so zu begrenzen, dass der Leistungswert einen vorbestimmten Mindest-Leistungswert nicht unterschreitet und/oder einen vorbestimmten Maximal-Leistungswert nicht überschreitet.

7. Hochfrequenz-Chirurgiegerät (1) nach mindestens einem der vorhergehenden Ansprüche,
**dadurch gekennzeichnet, dass** die Funkenkontrolleinrichtung (70) angeordnet und ausgebildet ist, den Soll-Funkenwert derart zu ermitteln, dass einem Mindest-Leistungswert ein bestimmter Soll-Funkenwert zugeordnet ist.

## Claims

1. High-frequency surgical device (1) for cutting and/or coagulating biological tissue,
- comprising a high-frequency generator (20) that is designed to generate a high-frequency alternating current during operation,
- comprising a power-setting device (60) that is arranged and designed to set and/or limit an output power of the high-frequency surgical device (1) to a power value selected by a user,
- comprising a spark control device (70) that is arranged and designed to set and/or limit a sparking voltage to a target sparking value,
- wherein the spark control device (70) is also designed to determine the target sparking value on the basis of the power value selected by a user, wherein the target sparking value is determined such that, at least in a specific value range, the target sparking value increases as the power value increases.

2. High-frequency surgical device (1) according to claim 1,
**characterised in that** the spark control device (70) is arranged and designed to determine the particular target sparking value such that, at least in a specific value range, the target sparking value increases proportionally to the power value.

3. High-frequency surgical device (1) according to at least one of the two preceding claims,
**characterised in that** the spark control device (70) is arranged and designed to determine the target sparking value such that the target sparking value does not fall below a predetermined minimum sparking value (100) and/or does not exceed a predetermined maximum sparking value.

4. High-frequency surgical device (1) according to at least one of the preceding claims,
**characterised in that** the high-frequency surgical device (1) comprises two, three or a plurality of operating modes (C1, C2, C3), and the spark control device (70) being designed and arranged to determine the target sparking value on the basis of the particular operating mode (C1, C2, C3).

5. High-frequency surgical device (1) according to the preceding claim,
**characterised in that** at least one of the operating modes comprises one, two, three or a plurality of effect modes, and the spark control device (70) being arranged and designed to determine the target sparking value on the basis of the particular effect mode.

6. High-frequency surgical device (1) according to at least one of the preceding claims,
**characterised in that** the power-setting device (60) is arranged and designed to limit the power value such that the power value does not fall below a predetermined minimum power value and/or does not exceed a predetermined maximum power value.

7. High-frequency surgical device (1) according to at least one of the preceding claims,
**characterised in that** the spark control device (70) is arranged and designed to determine the target sparking value such that a minimum power value is assigned to a specific target sparking value.

## Revendications

1. Instrument chirurgical haute fréquence (1), destiné à couper et/ou à faire coaguler un tissu biologique,
- avec un générateur haute fréquence (20) qui est conçu pour générer en service un courant alternatif haute fréquence,
- avec un système de réglage de la puissance (60) qui est placé et conçu pour régler, voire limiter une puissance de sortie de l'instrument chirurgical haute fréquence (1) à une valeur de puissance choisie par l'utilisateur,
- avec un système de contrôle des étincelles (70) qui est placé et conçu pour régler, voire limiter une tension d'étincelles à une valeur d'étincelle de consigne,
- le système de contrôle des étincelles (70) étant conçu en outre pour déterminer la valeur d'étincelle de consigne en fonction d'une valeur de puissance choisie par un utilisateur, la valeur d'étincelle de consigne étant déterminée de telle sorte que la valeur d'étincelle de consigne augmente au moins dans un certain ordre de valeurs au fur et à mesure de la montée de la valeur de puissance.

2. Instrument chirurgical haute fréquence (1) selon la revendication 1,
**caractérisé en ce que** le système de contrôle des étincelles (70) est placé et conçu pour déterminer la valeur d'étincelle de consigne respective, de sorte que la valeur d'étincelle de consigne augmente au moins dans un certain ordre de valeur proportionnellement à la valeur de puissance.

3. Instrument chirurgical haute fréquence (1) selon au moins l'une quelconque des deux revendications précédentes,
**caractérisé en ce que** le système de contrôle des étincelles (70) est placé et conçu pour déterminer la valeur d'étincelle de consigne de sorte que la valeur d'étincelle de consigne ne soit pas inférieure à une valeur d'étincelle minimale (100) prédéfinie et/ou ne dépasse pas une valeur d'étincelle maximale prédéfinie.

4. Instrument chirurgical haute fréquence (1) selon au moins l'une quelconque des revendications précédentes,
**caractérisé en ce que** l'instrument chirurgical haute fréquence (1) comporte deux, trois ou plusieurs modes de fonctionnement (C1, C2, C3) et le système de contrôle des étincelles (70) étant placé et conçu pour déterminer la valeur d'étincelle de consigne en fonction du mode de fonctionnement (C1, C2, C3) respectif.

5. Instrument chirurgical haute fréquence (1) selon la revendication précédente,
**caractérisé en ce qu'**au moins l'un des modes de fonctionnement comporte un, deux, trois ou plusieurs modes d'effet et le système de contrôle des étincelles (70) étant placé et conçu pour déterminer la valeur d'étincelle de consigne en fonction du mode d'effet respectif.

6. Instrument chirurgical haute fréquence (1) selon au moins l'une quelconque des revendications précédentes,
**caractérisé en ce que** le système de réglage de la puissance (60) est placé et conçu pour limiter la valeur de puissance de sorte que la valeur de puissance ne soit pas inférieure à une valeur de puissance minimale prédéterminée et/ou ne dépasse pas une valeur de puissance maximale prédéfinie.

7. Instrument chirurgical haute fréquence (1) selon au moins l'une quelconque des revendications précédentes,
**caractérisé en ce que** le système de contrôle des étincelles (70) est placé et conçu pour déterminer la valeur d'étincelle de consigne de sorte qu'à une valeur de puissance minimale soit associée une certaine valeur d'étincelle de consigne.
